# EUROPEAN PATENT SPECIFICATION

(11) **EP 3 291 818 B1**
(45) Date of publication and mention of the grant of the patent: **30.10.2019**
(21) Application number: 16789978.0
(22) Date of filing: 04.05.2016
(51) Int. Cl.: A61K 31/56, A61K 47/30, A61K 31/60, C08G 18/62, A61K 9/08, A61K 9/107

(54) **COMPOSITIONS AND METHODS FOR DELIVERING THERAPEUTIC AGENTS INTO THE COLON**
ZUSAMMENSETZUNGEN UND VERFAHREN ZUR ABGABE VON THERAPEUTIKA IN DEN DICKDARM
COMPOSITIONS ET PROCÉDÉS POUR L'ADMINISTRATION D'AGENTS THÉRAPEUTIQUES DANS LE CÔLON

(30) Priority: 04.05.2015 US 201562156682 P
(43) Date of publication of application: 14.03.2018
(73) Proprietor: The Board of Trustees of the Leland Stanford Junior University, Stanford, CA 94305-2038 (US)
(72) Inventor: SINHA, Sidhartha Ranjit, Stanford, California 94305-2038 (US); RAJADAS, Jayakumar, Stanford, California 94305-2038 (US); HABTEZION, Aida, Stanford, California 94305-2038 (US); PAMNANI, Ravinder D., Stanford, California 94305-2038 (US)
(74) Representative: Cabinet Laurent & Charras
(86) International application number: PCT/US2016/030682
(87) International publication number: WO 2016/179227

(56) References cited:
- US-A- 4 350 690
- US-A- 4 765 987
- US-A1- 2004 191 276
- US-A1- 2012 111 756
- KIM, DW ET AL.: 'The Influence of Bile Salt on the Chemotherapeutic Response of Docetaxel-Loaded Thermosensitive Nanomicelles.' INTERNATIONAL JOURNAL OF NANOMEDICINE. vol. 9, no. 1, 08 August 2014, pages 3816 - 3824, XP055329473
- CLINICALTRIALS.GOV: 'Efficacy and Safety of Budesonide Foam for Patients With Active Mild to Moderate Ulcerative Proctitis or Proctosigmoditis, NCT01008410.' CLINICAL TRIALS, [Online] November 2012, pages 1 - 6, XP055329474 Retrieved from the Internet: <URL:https://clinicaltrials.gov/ct2/show/NC T01008410?term=NCT01008410&rank=1> [retrieved on 2016-06-28]
- MOLOUGHNEY, JG ET AL.: 'Poloxamer 188 (P188) as a Membrane Resealing Reagent in Biomedical Applications.' RECENT PATENTS ON BIOTECHNOLOGY. vol. 6, no. 3, December 2012, pages 1 - 21, XP055209572

## Description

### FEDERALLY SPONSORED RESEARCH

This invention was made with Government support under contract DK007056 awarded by the National Institutes of Health. The Government has certain rights in the invention.

### FIELD OF THE INVENTION

The invention relates to enema compositions containing corticosteroids or salicylic acid derivatives. The compositions are liquid at room temperature but turn to gels at body temperature. They are useful for treating inflammatory bowel disease.

### BACKGROUND OF THE INVENTION

Inflammatory bowel disease (IBD) is a chronic, incurable disorder thought to be due to an exaggerated immune response to intestinal microbes in a genetically pre-disposed individual. About 1.5 million Americans suffer from inflammatory bowel disease (IBD) - either Crohn's disease (CD) or ulcerative colitis (UC) - and the prevalence is increasing globally. Both diseases are lifelong, relapsing illnesses that require induction followed by maintenance therapy. Patients with IBD often suffer many debilitating symptoms, such as bloody diarrhea and abdominal pain, and are at increased risk of colorectal cancer, particularly with uncontrolled disease.

Systemic and topical (applied directly to colonic mucosa) therapies exist. Current systemic therapies such as anti-TNF agents, immunomodulators, and steroids, while effective, have many known and potential side effects including increased risk of infections and malignancies. Topical therapies delivered rectally are recommended first line treatments, given their efficacy and fewer side effects when compared to systemic drugs. Topical therapies are effective in treating acute flares as well as maintaining remission for many patients. In fact, about half of IBD patients may benefit from topical therapy alone, since 66% of UC patients and 14% of CD patients have disease only in the distal colon and rectum, respectively.

The mainstay of topical therapy is the enema. Despite its advantages, patients with active distal colitis (those who would benefit most) are often unable to tolerate enemas due to urgency/spasm and the associated inability to retain a liquid solution for the recommended dosing and time frame (once or twice a day, retained for 1-3 hours). As a carrier for therapeutic agents, liquid enemas are effective at reaching all areas of the colon where inflammation may be present. However, liquid enemas are the most difficult for the patient to retain over time, given the near immediate urge to have a bowel movement once the liquid is present. Retention of the fluid is important in order to maximize the time for the therapeutic agent to diffuse from the carrier enema fluid to the mucosal wall of the digestive tract. The time span typically required is several hours, while the urge to evacuate one's bowels during a liquid enema is usually immediate (5-10 minutes). Alternatives to enemas for topical therapy include foam or suppository preparations. These options tend to be easier to retain but have the marked disadvantage of being unable to reach proximal areas of the left colon that are accessible with an enema. Despite the effectiveness of topical therapies, adherence remains extremely low due to inconvenience and discomfort associated with existing options. Current treatments for patients with IBD, while certainly beneficial, leave significant opportunity for improvement, particularly for topical therapies that are as effective as enemas for colitis involving the proximal colon, but are more tolerable and maintain low systemic side effects.

Reference is made to these prior art documents:
- US 2004/191276 A1
- KIM, DW ET AL.: "The Influence of Bile Salt on the Chemotherapeutic Response of Docetaxel-Loaded Thermosensitive Nanomicelles.", INTERNATIONAL JOURNAL OF NANOMEDICINE., vol. 9, no. 1, 8 August 2014 (2014-08-08), pages 3816-3824, XP055329473.

### SUMMARY OF THE INVENTION

In one aspect, the invention relates to an enema composition comprising:
a. a non-ionic block copolymer or mixture of polymers consisting of polyethylene glycol and polypropylene glycol blocks;
b. a phospholipid or mixture of phospholipids;
c. a corticosteroid; and
d. water;
wherein the concentration of the non-ionic block copolymer or mixture of polymers is from 200 to 400 g/L; the concentration of the phospholipid or mixture of phospholipids is from 0.04 to 4 g/L; the concentration of the corticosteroid is from 0.05 to 5 g/L (all based on total composition); and the remainder of the volume comprises water;
such that a gel comprising components a-d exhibits a gel transition temperature between 32 and 38 degrees C.

In a second aspect, the invention relates to enema composition comprising:
a. a non-ionic block copolymer or mixture of polymers consisting of polyethylene glycol and polypropylene glycol blocks;
b. a phospholipid or mixture of phospholipids;
c. a salicylic acid derivative; and
d. water;
wherein the concentration of said non-ionic block copolymer or mixture of polymers is from 100 to 300 g/L of said composition; the concentration of said phospholipid or mixture of phospholipids is from 4 to 40 g/L; the concentration of said salicylic acid derivative is from 50 to 100 g/L; and the remainder of the volume comprises water;
such that a gel comprising said components a-d exhibits a gel transition temperature between 32 and 38 degrees C.

In another aspect, the invention relates to a method for treating inflammatory bowel disease comprising administering an enema composition as described above.

### DETAILED DESCRIPTION OF THE INVENTION

Given the known issues with retention of liquid enemas and the limitations of suppositories and foams, we have developed a thermosensitive enema composition that has the advantages of liquid enema (more proximal delivery) but without retention issues associated with liquids. Briefly, this is accomplished by a composition that is a liquid at cooler temperatures, near room temperature (23°C), and then transitions into a viscous gel at closer to body temperature (37°C). One component of the composition is a non-ionic surfactant copolymer consisting of hydrophilic polyethylene glycol and hydrophobic polypropylene glycol blocks. As temperature increases, the copolymers form micelles and at higher temperatures, a polymer gel matrix is formed.

The enema composition described above in some embodiments contains either a triblock copolymer of polyethylene glycol and polypropylene glycol having a polyoxypropylene molecular mass of about 4,000 g/mol and about 70% polyoxyethylene content or having a polyoxypropylene molecular mass of about 1,800 g/mol and about 80% polyoxyethylene content. Commercially available examples of such PEO/PPO block copolymers are

| Poloxamer | Average Molecular Weight | Weight % Oxyethylene | Unsaturation,mEq/g |
|---|---|---|---|
| 124 | 2090 to 2360 | 46.7±1.9 | 0.020±0.008 |
| 188 | 7680 to 9510 | 81.8±1.9 | 0.026±0.008 |
| 237 | 6840 to 8830 | 72.4±1.9 | 0.034±0.008 |
| 338 | 12700 to 17400 | 83.1±1.7 | 0.031±0.008 |
| 407 | 9840 to 14600 | 73.2±1.7 | 0.048±0.017 |

The commercially available materials may additionally contain a suitable antioxidant. The foregoing chart is taken from *http:*//*www.new druginfo.com*/*pharmacopeia*/*usp28*/*v28230*/*usp28nf23s0_m66210.htm*. Because the copolymers are complex mixtures, they are usually described as having an average molecular weight of "about" x or a molecular mass of "about" y g/mol or a PEG content of "about" 70%. Persons of skill in the art recognize that greater precision is simply not practical, and the use of the term "about" in this context is not indefinite. If a numerical value must be assigned, most persons of skill would probably be comfortable (based on the figures above) with ± 40% on the average molecular weight or molecular mass, and ± 5% on the percentage of monomer content. Poloxamer 407 is a triblock (PEO-PPO-PEO) copolymer with an average molecular weight of about 12.5 KDa and a PEO/PPO ratio of about 2:1 (Bohorquez et al., J Colloid Interface Sci 1999; 216:34-40).

In the most convenient embodiments, i.e. those not requiring refrigeration, the lower practical limit for a transition temperature is about 25°C, with other transition temperatures of 26°C, 27°C, 28°C, 29°C, 30°C, 31°C, 32°C, 33°C, 34°C, 35°C or 36°C being possible. Optimally, the composition transitions to a viscous gel near 37° C. The transition temperature for gel formation appears to be somewhat dependent on the measurement system used to determine it. Oscillatory measurement (e.g. HAAKE RheoStress 6000, Thermo Fisher Scientific) provides a slightly lower temperature and sharper curve than rotational methods, because rotational testing can raise the apparent transition temperature if the rotation disrupts the gel formation. We have used a parallel plate rheometer for our studies, but the temperatures defining the gel transition for the purpose of the claims should be determined by oscillatory measurement. The sol-gel transition temperature is determined by making oscillation measurements at 1 rad.s-1 while the temperature is increased at 2°C.min-1. The sol-gel transition temperature is determined by plotting the storage modulus (G') as a function of temperature. For the purpose of the instant invention, we will define the gel transition temperature as the temperature at which the slope of a curve of storage modulus versus temperature, measured at 1 Hz, exceeds 5 kPa per degree Celsius.

In some embodiments, the enema composition comprises from 100 to 400 g/L of copolymer. In other embodiments, the enema composition comprises from 100 to 300 g/L of the copolymer. In other embodiments, the enema composition comprises from 200-400 g/L, 150-250 g/L, 250-350 g/L, 100-200 g/L, 200-300 g/L, 300-400 g/L, 100-150 g/L, 150-200 g/L, 200-250 g/L, 250-300 g/L, 300-350 g/L or 350-400 g/L.

In some embodiments the phospholipid or mixture of phospholipids is one or both of dipalmitoylphosphatidylcholine (DPPC) and 1,2-distearoyl-sn-glycero-3-phosphocholine (DSPC). Preferred phospholipids are glycerophospholipids, such as phosphatidic acid, phosphatidylethanolamine, phosphatidylcholine (lecithins), phosphatidylserine, and phosphoinositides. Phosphatidylcholines are particularly preferred.

In some embodiments, the corticosteroid is chosen from budesonide, dexamethasone, hydrocortisone, methylprednisolone, prednisolone, and prednisone. In some the corticosteroid is chosen from budesonide and hydrocortisone. In the examples below, the corticosteroid is budesonide.

In an embodiment, the enema composition is administered at less than 30 degrees C. In another embodiment, the enema composition is administered at less than 25 degrees C.

In a very specific embodiment, the enema composition consists essentially of:
a. from 250 to 350g/L of a triblock copolymer of polyethylene glycol and polypropylene glycol having a polyoxypropylene molecular mass of about 4,000 g/mol and about 70% polyoxyethylene content.;
b. from 0.1 to 1 g/L of a 1:1 mixture of dipalmitoylphosphatidylcholine (DPPC) and 1,2-distearoyl-sn-glycero-3-phosphocholine (DSPC);
c. from 0.05 to 0.2 g/L of budesonide; and
d. the remainder, water.

In some embodiments, the salicylic acid derivative is chosen from mesalazine, sulfasalazine, olsalazine, and balsalazide. Mesalazine is preferred. Mesalazine (INN, BAN) is also known as mesalamine (USAN) or 5-aminosalicylic acid (5-ASA).

In a very specific embodiment, the enema composition consists essentially of:
a. from 150 to 250 g/L of a triblock copolymer of polyethylene glycol and polypropylene glycol having a polyoxypropylene molecular mass of about 4,000 g/mol and about 70% polyoxyethylene content.;
b. from 5 to 20 g/L of a 1:1 mixture of dipalmitoylphosphatidylcholine (DPPC) and 1,2-distearoyl-sn-glycero-3-phosphocholine (DSPC);
c. from 60 to 80 g/L of mesalazine; and
d. the remainder, water.

Since the compositions of the invention are gels, they are free of propellants (e.g. hydrocarbon gases) and free of ingredients that are necessary for foams but unnecessary for gels. Typically the compositions described herein are free of foam stabilizers and foaming agents such as sodium lauryl sulphate, lauric acid, myristic acid, palmitic acid, stearic acid, coconut oil, carrageenan, stearic monoethonolamine, gum tragacanth, alginate, gelatin, sodium CMC, polyvinyl glycol, glycerol, sorbitol, hydrogenated castor oil, polysorbate 20, cocamidopropyl betaine, and caprylic/ capric glycerides. The greater comfort and the ability to accommodate larger volume compared to other modalities may additionally provide for a less frequent dosing schedule.

To test the ability of the enema compositions to be retained in the colon, 3% methylene blue was added to a formulation as described herein, and enemas were administered to C57BL/6 mice. Compositions embodying the invention containing methylene blue were clearly better retained than methylene blue in conventional liquid enema solutions.

A composition with budesonide (referenced as "BPL" for budesonide, polymer, lipid) was prepared according to the invention: Contents: Budesonide 10mg (0. 1mg/mL), Poloxamer 407 30g (30% solution), DSPC 20mg (0.2 mg/mL) and DPPC 20mg (0.2 mg/mL). Method: DSPC (1,2-Distearoyl-sn-glycero-3-phosphocholine) (20 mg), DPPC (1,2-dipalmitoyl-sn-glycero-3-phosphocholine)(20mg) and budesonide (10mg) were dissolved in 5 mL of ethanol in a round bottom flask. The solvent was evaporated in a rotary evaporator to form a thin film in the inner surface of flask. The film was suspended in water (20 mL) and sonicated for 30 minutes to obtain a liposome solution. To this 30 g of Poloxamer was added and the volume was made up to 100mL with water at 4°C. This was stirred for 30 minutes, the trapped air bubbles were removed by centrifuging, and the stirring was continued till a homogeneous solution was obtained. Budesonide was dissolved in enough solution to obtain a 0.1mg/mL concentration.

A composition with mesalazine (referenced as "MPL" for mesalazine, polymer, lipid) was also prepared. Method: Phospholipids 1,2-Distearoyl-sn-glycero-3-phosphocholine (DSPC) (50 mg), (CordenPharma), 1,2-Dipalmitoyl-sn-glycero-3-phosphocholine (DPPC) (50 mg), (CordenPharma), and mesalazine (667 mg), (AK Scientific), were dissolved in 30 mL of ethanol in a round bottom flask. The ethanol was evaporated in a rotary evaporator to form a thin film in the inner surface of a flask. The film was suspended in water (3.5 mL) and sonicated for 30 minutes to obtain a homogeneous liposomal solution. A solution containing 2g of poloxamer in 5 mL of water was then added to the liposomes at 4°C. The mixture was stirred for 30 minutes and the trapped air bubbles were removed by centrifuging at 600 × g in Heraeus Labofuge - 400 centrifuge. Water (∼1 mL) was added at 4°C to make the final volume 10mL to obtain the concentration 67 mg/mL. The stirring was continued at 4°C until a homogeneous solution (mesalazine-poloxamer-lipid, MPL) was obtained. The final solution contained mesalazine (67 mg/mL), phospholipids (10 mg/mL) and poloxamer (20% w/v) in water.

Phospholipids 1,2-Distearoyl-sn-glycero-3-phosphocholine (DSPC) (50 mg), (CordenPharma), 1,2-Dipalmitoyl-sn-glycero-3-phosphocholine (DPPC) (50 mg), (CordenPharma), and mesalazine (667 mg), (AK Scientific), were dissolved in 30 mL of ethanol in a round bottom flask. The ethanol was evaporated in a rotary evaporator to form a thin film in the inner surface of a flask. The film was suspended in water (3.5 mL) and sonicated for 30 minutes to obtain a homogeneous liposomal solution. A solution containing 2g of poloxamer in 5 mL of water was then added to the liposomes at 4°C. The mixture was stirred for 30 minutes and the trapped air bubbles were removed by centrifuging at 600 × g in Heraeus Labofuge - 400 centrifuge. Water (∼1 mL) was added at 4°C to make the final volume 10mL to obtain the concentration 67 mg/mL. The stirring was continued at 4°C until a homogeneous solution (mesalazine-poloxamer-lipid, MPL) was obtained. The final solution contained mesalazine (67 mg/mL), phospholipids (10 mg/mL) and poloxamer (20% w/v) in water.

Another mesalazine composition with 6.6% mesalazine in 60 mL total volume was also prepared. Method: Ten grams of Poloxamer 407 (0.79 mmol) was melted at 60°C neat and 0.3 g of DSPC (0.77 mmol) was added followed by 0.3 g of DPPC (0.4 mmol). The mixture was stirred and 4 g of 5-aminosalicylic acid (26.12 mmol) was added. The mixture was stirred with mechanical stirring for approximately 1 hour, then cooled to 4°C. Sixty mL of deionized water was added and the mixture was stirred at 4°C overnight. The resulting liquid exhibited a transition temperature of 32-33°C.

The foregoing formulation can be expanded to a 100 mL total volume with the same mass of total drug (4g for mesalazine, 6 mg for budesonide). The concentrations are, of course, lower in the 100-mL volume solutions, but are still effective. For mesalazine, the concentration would be 40 mg/mL, and for budesonide, this would be 0.06 mg/mL.

We conducted animal studies of BPL in a well-established colitis model, dextran sulphate sodium (DSS) (orally administered) model, in C57BL/6 mice. DSS was added to the drinking water and given daily starting on Day 0. The mice were given enema treatments on Day 4 and Day 6. Daily weights were recorded, as weight is one of the most commonly used metrics to determine treatment response in colitis models. The BPL group demonstrated improvement versus water and polymer controls, and also over budesonide liquid (BL), as evidenced by less weight loss compared to original body weight. These results were replicated in two additional experiments in a total of fifty mice. In addition to weight, we also measured colon length after treatment for all mice. Mice treated with BPL had longer colons (with well formed stool pellets) compared to other treatment and control groups. Histopathology of sections of explanted mice colons showed reduced leukocyte infiltrate and more preserved epithelial architecture in BPL mice compared to other groups.

Next we conducted experiments to establish the retention kinetics and proximal distribution of BPL compared to a standard liquid treatment enema (BL). To confirm that enemas with BPL will have similar proximal distribution as BL, we gave standardized enemas with barium contrast to both healthy and DSS-induced colitis mice. The mice were then imaged with computerized tomography at pre-determined intervals (at 0.5, 1.5, and 3 hours) to evaluate maximum retrograde distance reached by the enema. BPL was shown to actually have better retrograde distribution than BL in both healthy mice and mice with colitis. In addition, in order to determine the possible mucoadhesiveness of BPL versus BL we utilized 3-D imaging software to assess the volume of enema retained at the same pre-determined intervals. Again, the volume of enema retained at all time points measured was substantially greater for BPL than BL in both healthy mice and mice with colitis.

Similar to the experiments with BPL, the MPL formulation demonstrated improvement versus water and polymer controls and also over mesalazine liquid (ML) as evidenced by less weight loss compared to original body weight. These results were replicated in an additional experiment with a total of 33 mice. Mice treated with MPL had longer colons compared to other treatment and control groups. Histopathology of sections of explanted mice colons showed healthier mucosa in MPL mice compared to other groups.

To further test the robustness of the therapy in treating colitis, another commonly used IBD colitis model--trinitrobenzene sulfonic acid (TNBS)-induced acute colitis modelwas also used to test the compositions described herein in Balb/c mice. We tested BPL vs. BL and water control. Again, the BPL group regained weight faster than the other two groups after receiving TNBS enema followed by a single treatment or water enema. This experiment was repeated with consistent results.

It should be understood that in addition to the ingredients particularly mentioned above, the formulations of this invention may include other agents conventional in the art of pharmaceutical gels.

As used herein, the terms "treatment" or "treating," or "palliating" or "ameliorating" refer to an approach for obtaining beneficial or desired results including but not limited to therapeutic benefit and/or a prophylactic benefit. A therapeutic benefit is achieved with the eradication or amelioration of one or more of the physiological symptoms associated with the underlying disorder such that an improvement is observed in the patient, notwithstanding that the patient may still be afflicted with the underlying disorder. The compositions may be administered to a patient reporting one or more of the physiological systems of Inflammatory Bowel Disease, even though a diagnosis of this disease may not have been made.

### Materials and Methods

C57BL/6 mice were purchased from Taconic (Hudson, NY) and housed for one week before experiments. Balb/c mice were purchased from Jackson Laboratory (Bar Harbor, ME) and bred in house for TNBS colitis studies. Care and use of animals were in accordance to National Institutes of Health guidelines and approved by Stanford University institutional animal care and use committees.

The shear modulus and viscosity of copolymer thermogels were measured with an Ares® rheometer (Rheometric Scientific®) equipped with an oven for precise temperature control in a parallel plate geometry. Frequency scan measurements (0.5 - 200 rad/s) were performed for every temperature, while maintaining a constant strain of 1%.

C57BL/6 mice were given 2% (w/v) dextran sulfate sodium salt (36,000-50,000 M.W.; MP Biomedicals, LLC; Solon, OH) in drinking water for the duration of the experiment. Mice were weighed daily and assessed for presence of bloody stool, diarrhea and general well being. Treatment (BPL, BL) and control (water, polymer only) enemas were given on day 4 and day 6. Isoflorane-anesthetized mice were rectally given 150 µL of indicated solution through a 1 mL Luer Lock syringe attached to 23G needle and polyethylene tubing (0.048 O.D. in), with tubing inserted ∼2 cm. Animals were sacrificed on day 9 or earlier if moribund or their weight loss exceeded 20%.

Balb/c mice were anesthetized with ketamine and rectally treated with 2 mg picrylsulfonic acid (2,4,6-trinitrobenzenesulfonic acid solution; Sigma-Aldrich; St. Louis, MO) in 100 µL volume, as previously described to induce TNBS colitis. Control mice received 100 µL of vehicle (40% ethanol). The next day (day 1) mice were anesthetized with isoflurane and given treatment or control enemas of 150 µL, as with DSS colitis model. Animals were weighed and assessed daily and sacrificed at or before loss of 20% body weight.

For histological assessment, the most-distal 1 cm colon segments were fixed in 10% formalin-buffered, paraffin-embedded and sectioned for hematoxylin and eosin staining. Histopathology was scored in blinded fashion as described by Ostanin et al. "T cell transfer model of chronic colitis: concepts, considerations, and tricks of the trade." Am. J. Physiology. Gastrointestinal and liver physiology 296, G135-146 (2009*).*

Statistical analyses were done using Prism software (GraphPad Software; San Diego, CA) and statistical tests employed are indicated in the figures, with p-values of <0.05 considered as significant.

For CT imaging C57BL/6 mice were given water containing 2% (w/v) DSS and on day 6 (when bloody stool is apparent) were administered 150 µL BPL or BL enemas containing 5% (v/v) barium sulfate suspension (E-Z-EM, Inc.; Lake Success, NY). The enema tubing was inserted in a standardized manner with insertion between 0.5 to 1.0 cm. The animals were imaged in the Inveon PET-CT with large field CT scanner (Siemens Medical Solutions USA, Inc.; Hoffman Estates, IL) at the Stanford Center for Innovation in In-Vivo Imaging (Stanford, CA). Retrograde distance and enema volume were calculated.

Animal studies suggest that topically applied inhibitors of tumor necrosis factor (TNF)-α ameliorate colitis severity in animal models. Suitable concentrations of (TNF)-α could be formulated analogously to those for budesonide and mesalazine.

Although this invention is susceptible to embodiment in many different forms, preferred embodiments of the invention are shown. It should be understood, however, that the present disclosure is to be considered as an exemplification of the principles of this invention and is not intended to limit the invention to the embodiments illustrated.

## Claims

1. An enema composition comprising:
a. a non-ionic block copolymer or mixture of polymers consisting of polyethylene glycol and polypropylene glycol blocks;
b. a phospholipid or mixture of phospholipids;
c. a corticosteroid; and
d. water;
wherein
the concentration of said non-ionic block copolymer or mixture of polymers is from 200 to 400 g/L, advantageously from 250 to 350g/L;
the concentration of said phospholipid or mixture of phospholipids is from 0.04 to 4 g/L; the concentration of said corticosteroid is from 0.05 to 5 g/L;
and the remainder of the volume comprises water;
such that a gel comprising said components a-d exhibits a gel transition temperature between 32 and 38 degrees C.

2. An enema composition comprising:
a. a non-ionic block copolymer or mixture of polymers consisting of polyethylene glycol and polypropylene glycol blocks;
b. a phospholipid or mixture of phospholipids;
c. a salicylic acid derivative; and
d. water;
wherein
the concentration of said non-ionic block copolymer or mixture of polymers is from 100 to 300 g/L, advantageously from 150 to 250 g/L;
the concentration of said phospholipid or mixture of phospholipids is from 4 to 40 g/L;
the concentration of said salicylic acid derivative is from 50 to 100 g/L;
and the remainder of the volume comprises water;
such that a gel comprising said components a-d exhibits a gel transition temperature between 32 and 38 degrees C.

3. An enema composition according to either of claims 1 or 2 wherein at least one said block copolymer is a triblock copolymer of polyethylene glycol and polypropylene glycol having a polyoxypropylene molecular mass of about 4,000 g/mol and about 70% polyoxyethylene content.

4. An enema composition according to either of claims 1 or 2 wherein at least one said block copolymer is a triblock copolymer of polyethylene glycol and polypropylene glycol having a polyoxypropylene molecular mass of about 1,800 g/mol and about 80% polyoxyethylene content.

5. An enema composition according to claim 1 or 2 wherein said phospholipid or mixture of phospholipids is one or both of dipalmitoylphosphatidylcholine (DPPC) and 1,2-distearoyl-sn-glycero-3-phosphocholine (DSPC).

6. An enema composition according to claim 1 wherein said corticosteroid is chosen from budesonide, dexamethasone, hydrocortisone, methylprednisolone, prednisolone, and prednisone, advantageously from budesonide and hydrocortisone.

7. An enema composition according to claim 6 wherein said corticosteroid is budesonide.

8. An enema composition according to claim 7 consisting of:
a. from 250 to 350g/L of a triblock copolymer of polyethylene glycol and polypropylene glycol having a polyoxypropylene molecular mass of about 4,000 g/mol and about 70% polyoxyethylene content.;
b. from 0.1 to 1 g/L of a 1:1 mixture of dipalmitoylphosphatidylcholine (DPPC) and 1,2-distearoyl-sn-glycero-3-phosphocholine (DSPC);
c. from 0.05 to 0.2 g/L of budesonide; and
d. the remainder, water.

9. An enema composition according to claim 2 wherein said salicylic acid derivative is chosen from mesalazine, sulfasalazine, olsalazine, and balsalazide.

10. An enema composition according to claim 9 wherein said salicylic acid derivative is mesalazine.

11. An enema composition according to claim 10 consisting of:
a. from 150 to 250 g/L of a triblock copolymer of polyethylene glycol and polypropylene glycol having a polyoxypropylene molecular mass of about 4,000 g/mol and about 70% polyoxyethylene content.;
b. from 5 to 20 g/L of a 1:1 mixture of dipalmitoylphosphatidylcholine (DPPC) and 1,2-distearoyl-sn-glycero-3-phosphocholine (DSPC);
c. from 60 to 80 g/L of mesalazine; and
d. the remainder, water.

12. An enema composition according to claim 8 or 11 of 60 mL to 100 mL total volume.

13. An enema composition according to claim 1 or 2 for use in treating inflammatory bowel disease.

14. A composition for its use according to claim 13 wherein said enema composition is administered at a temperature below the gel temperature of the composition.

15. A composition for its use according to claim 14 wherein said enema composition is at less than 30 degrees C when administered.

## Patentansprüche

1. Eine Einlaufzusammensetzung, die enthält:
a. ein nicht-ionisches Block-Copolymer oder eine Polymer-Mischung, die aus Polyethylenglycol- und Polypropylenglycol-blöcken besteht;
b. ein Phospholipid oder eine Mischung aus Phospholipiden;
c. ein Corticosteroid; und
d. Wasser; wobei
die Konzentration des genannten nicht-ionischen Block-Copolymers oder der Polymer-Mischung, zwischen 200 und 400 g/L, besser noch zwischen 250 und 350g/L liegt;
die Konzentration des genannten Phospholipids oder der Mischung aus Phospholipiden zwischen 0,04 und 4 g/L liegt;
die Konzentration des genannten Corticosteroids zwischen 0,05 und 5 g/L liegt; und der Rest des Volumens aus Wasser besteht;
so dass ein Gel aus diesen Komponenten a-d eine Gel-Übergangstemperatur zwischen 32 und 38 Grad C aufweist.

2. Einlaufzusammensetzungen, die enthält:
a. ein nicht-ionisches Block-Copolymer oder eine Polymer-Mischung, die aus Polyethylenglycol und Polypropylenglycolblöcken besteht;
b. Phospholipid oder Mischung aus Phospholipiden;
c. ein Salicylsäurederivat; und
d. Wasser;
wobei
die Konzentration des genannten nicht-ionisches Block-Copolymer oder der Polymer-Mischung, zwischen 100 und 300 g/L, besser noch zwischen 150 und 250 g/L liegt;
die Konzentration des genannten Phospholipids oder der Mischung aus Phospholipiden zwischen 4 und 40 g/L liegt;
die Konzentration des genannten Salicylsäurederivats zwischen 50 und 100 g/L liegt und der Rest des Volumens aus Wasser besteht;
so dass ein Gel aus diesen Komponenten a-d eine Gel-Übergangstemperatur zwischen 32 und 38 Grad C aufweist.

3. Einlaufzusammensetzung gemäß Anspruch 1 oder 2, wobei mindestens das eine genannte Block-Copolymer ein Triblock-Copolymer aus Polyethylenglycol und Polypropylenglycol, mit einer Polyoxypropylen- Molekülmasse von ungefähr 4.000 g/mol und ungefähr 70 % Polyoxyethylen-Inhalt, ist.

4. Einlaufzusammensetzung gemäß Anspruch 1 oder 2, wobei mindestens das eine genannte Block-Copolymer ein TriBlock-Copolymer aus Polyethylenglycol und Polypropylenglycol, mit einer Polyoxypropylen- Molekülmasse von ungefähr 1.800 g/mol und ungefähr 80 % Polyoxyethylen-Inhalt, ist.

5. Einlaufzusammensetzung gemäß Anspruch 1 oder 2, wobei es sich bei diesem Phospholipid oder der Mischung aus Phospholipiden um Dipalmitoylphosphatidylcholin (DPPC) oder 1,2-distearoyl-sn-glycero-3-phosphocholin (DSPC) oder um beide handelt.

6. Einlaufzusammensetzung gemäß Anspruch 1, wobei das genannte Corticosteroid aus Budesonid, Dexamethason, Hydrocortison, Methylprednisolon, Prednisolon und Prednison, am besten aus Budesonid und Hydrocortison ausgewählt wird.

7. Einlaufzusammensetzung gemäß Anspruch 6, wobei das genannte Corticosteroid Budesonid ist.

8. Einlaufzusammensetzung gemäß Anspruch 7, bestehend aus:
a. zwischen 250 und 350g/L eines TriBlock-Copolymers aus Polyethylenglycol und Polypropylenglycol mit einer Polyoxypropylen-Molekülmasse von ungefähr 4.000 g/mol und ungefähr 70 % Polyoxyethylen-Inhalt;
b. zwischen 0,1 und 1 g/L einer 1:1 Mischung aus Dipalmitoylphosphatidylcholin (DPPC) und 1,2-distearoyl-sn-glycero-3-phosphocholin (DSPC);
c. zwischen 0,05 und 0,2 g/L Budesonid; und
d. dem Rest, Wasser.

9. Einlaufzusammensetzung gemäß Anspruch 2, wobei das genannte Salicylsäurederivat aus Mesalazin, Sulfasalazin, Olsalazin und Balsalazid ausgewählt wird.

10. Einlaufzusammensetzung gemäß Anspruch 9, wobei das genannte Salicylsäurederivat Mesalazin ist.

11. Einlaufzusammensetzung gemäß Anspruch 10, bestehend aus:
a. zwischen 150 und 250 g/L eines TriBlock-Copolymers aus Polyethylenglycol und Polypropylenglycol mit einer Polyoxypropylen-Molekülmasse von ungefähr 4.000 g/mol und ungefähr 70 % Polyoxyethylen-Inhalt;
b. zwischen 5 und 20 g/L einer 1:1 Mischung aus Dipalmitoylphosphatidylcholin (DPPC) und 1,2-distearoyl-sn-glycero-3-phosphocholin (DSPC);
c. zwischen 0,05 und 80 g/L Mesalazin; und
d. dem Rest, Wasser.

12. Einlaufzusammensetzung gemäß Anspruch 8 oder 11 mit 60 ml bis 100 ml Gesamtvolumen.

13. Einlaufzusammensetzung gemäß Anspruch 1 oder 2, zur Verwendung in der Behandlung entzündlicher Darmerkrankungen.

14. Einlaufzusammensetzung zur Verwendung gemäß Anspruch 13, wobei die genannte Einlaufzusammensetzung mit einer Temperatur unter der Geltemperatur der Zusammensetzung verabreicht wird.

15. Einlaufzusammensetzung zur Verwendung gemäß Anspruch 14, wobei die genannte Einlaufzusammensetzung bei der Verabreichung eine Temperatur von unter 30 Grad C hat.

## Revendications

1. Composition de lavement comprenant:
a. un copolymère bloc non-ionique ou un mélange de polymères constitué de blocs de polyéthylène glycol et de polypropylène glycol;
b. un phospholipide ou un mélange de phospholipides;
c. un corticostéroïde; et
d. de l'eau;
dans laquelle
la concentration dudit copolymère bloc non-ionique ou du mélange de polymères est comprise entre 200 et 400 g/L, avantageusement entre 250 et 350 g/L ;
la concentration dudit phospholipide ou du mélange de phospholipides est comprise entre 0.04 et 4 g/L ;
la concentration dudit corticostéroïde est comprise entre 0.05 et 5 g/L ;
et le reste du volume comprend de l'eau ;
de sorte qu'un gel comprenant lesdits composés a-d présente une température de transition vitreuse comprise entre 32 et 38°C.

2. Composition de lavement comprenant :
a. un copolymère bloc non-ionique ou un mélange de polymères constitué de blocs de polyéthylène glycol et de polypropylène glycol;
b. un phospholipide ou un mélange de phospholipides;
c. un dérivé d'acide salicylique; et
d. de l'eau;
dans laquelle
la concentration dudit copolymère bloc non-ionique ou le mélange de polymères est compris entre 100 et 300 g/L, avantageusement entre 150 et 250 g/L ;
la concentration dudit phospholipide ou du mélange de phospholipides est comprise entre 4 et 40 g/L ;
la concentration dudit dérivé d'acide salicylique est comprise entre 50 et 100 g/L ;
et le reste du volume comprend de l'eau ;
de sorte qu'un gel comprenant lesdits composés a-d présente une température de transition vitreuse comprise entre 32 et 38°C.

3. Composition de lavement selon l'une des revendications 1 ou 2 dans laquelle au moins un dudit copolymère bloc est un copolymère tri-bloc de polyéthylène glycol et de polypropylène glycol comprenant un polyoxypropylène de masse moléculaire d'environ 4000 g/mol et contenant environ 70% de polyoxyéthylène.

4. Composition de lavement selon l'une des revendications 1 ou 2 dans laquelle au moins un dudit copolymère bloc est un copolymère tri-bloc de polyéthylène glycol et de polypropylène glycol comprenant un polyoxypropylène de masse moléculaire d'environ 1800 g/mol et contenant environ 80% de polyoxyéthylène.

5. Composition de lavement selon l'une des revendications 1 ou 2 dans laquelle ledit phospholipide ou mélange de phospholipides est la dipalmitoylphosphatidylcholine (DPPC) et/ou la 1, 2-distéaroyl-sn-glycéro-3-phosphocholine (DSPC).

6. Composition de lavement selon la revendication 1 dans laquelle ledit corticostéroïde est sélectionné parmi la budénoside, la dexaméthasone, l'hydrocortisone, la méthylprednisolone, la prednisolone et la prednisone, avantageusement parmi la budénoside et l'hydrocortisone.

7. Composition de lavement selon la revendication 6 dans laquelle ledit corticostéroïde est la budésonide.

8. Composition de lavement selon la revendication 7 constituée de :
a. 250 à 350 g/L d'un copolymère tri-bloc de polyéthylène glycol et de polypropylène glycol comprenant un polyoxypropylène de masse moléculaire d'environ 4000 g/mol et contenant environ 70% de polyoxyéthylène ;
b. 0.1 à 1 g/L d'un mélange 1:1 de dipalmitoylphosphatidylcholine (DPPC) et de 1, 2-distéaroyl-sn-glycéro-3-phosphocholine (DSPC) ;
c. 0.05 à 0.2 g/L de budésonide; et
d. le reste étant de l'eau.

9. Composition de lavement selon la revendication 2 dans laquelle ledit dérivé d'acide salicylique est sélectionné parmi la mésalazine, la sulfasalazine, l'olsalazine et la balsalazide.

10. Composition de lavement selon la revendication 9 dans laquelle ledit dérivé d'acide salicylique est de la mésalazine.

11. Composition de lavement selon la revendication 10 constituée de:
a. 150 à 250 g/L d'un copolymère tri-bloc de polyéthylène glycol et de polypropylène glycol comprenant un polyoxypropylène de masse moléculaire d'environ 4000 g/mol et contenant environ 70% de polyoxyéthylène ;
b. 5 à 20 g/L d'un mélange 1:1 de dipalmitoylphosphatidylcholine (DPPC) et de 1, 2-distéaroyl-sn-glycéro-3-phosphocholine (DSPC);
c. 60 à 80 g/L de mésalazine; et
d. le reste étant de l'eau.

12. Composition de lavement selon la revendication 8 ou 11 ayant un volume total de 60 mL à 100 mL.

13. Composition de lavement selon la revendication 1 ou 2 pour utilisation dans le traitement des pathologies liées à l'inflammation de l'intestin.

14. Composition pour son utilisation selon la revendication 13, selon laquelle ladite composition de lavement est administrée à une température inférieure à la température de transition vitreuse de la composition.

15. Composition pour son utilisation selon la revendication 14, selon laquelle ladite composition de lavement est à moins de 30 °C lorsqu'elle est administrée.
